# FASCICULE DE BREVET EUROPEEN

(11) **EP 0 586 303 B1**
(45) Date de publication et mention de la délivrance du brevet: **02.10.1996**
(21) Numéro de dépôt: 93402134.6
(22) Date de dépôt: 01.09.1993
(51) Int. Cl.: A61K 7/40, A61K 7/48, A61K 7/06, A61K 7/28, A61K 38/44

(54) **Composition cosmétique ou pharmaceutique comprenant en association une peroxydase et un agent anti-oxygène singulet**
Kosmetische oder pharmazeutische Zusammensetzung enthaltend eine Kombination einer Peroxydase mit einem Mittel gegen Singulett-Sauerstoff
Cosmetic or pharmaceutical composition containing an association between a peroxydase and an anti-singlet oxygen agent

(30) Priorité: 01.09.1992 FR 9210439
(43) Date de publication de la demande: 09.03.1994
(73) Titulaire: L'OREAL, F-75008 Paris (FR)
(72) Inventeur: N'Guyen, Quang Lan, F-92160 Antony (FR); Colin, Christian, F-75020 Paris (FR)
(74) Mandataire: Tonnellier, Jean-Claude

(56) Documents cités:
- WO-A-88/02600
- WO-A-92/01466
- FR-A- 2 112 550
- US-A- 4 473 550
- DATABASE WPI Week 8943, Derwent Publications Ltd., London, GB; AN 89-310020 & DD-A-268 157 (VEB BERLIN-KOSMETIK) 24 Mai 1989

## Description

L'invention a pour objet une composition cosmétique ou pharmaceutique à action synergique contenant en association une peroxydase et un antioxydant.

Les spécialistes considèrent actuellement qu'une des causes du vieillissement cellulaire est l'amoindrissement des capacités de défense contre les radicaux libres et contre les phénomènes d'oxydation (notamment la formation de peroxydes) qu'ils initient.

On sait par ailleurs que la toxicité des polluants atmosphériques, notamment des polluants gazeux tels que le dioxyde de soufre, l'ozone et les oxydes d'azote, est liée notamment à leur activité d'initiateurs de radicaux libres, source de phénomènes d'oxydation qui provoquent chez les êtres vivants des dommages cellulaires.

Les cellules vivantes, qui sont en contact direct et permanent avec le milieu extérieur (notamment la peau, le cuir chevelu et certaines muqueuses), sont particulièrement sensibles à ces effets des polluants gazeux, qui se traduisent notamment par un vieillissement accéléré de la peau, avec un teint manquant d'éclat et une formation précoce de rides ou ridules, et aussi par une diminution de la vigueur et un aspect terne des cheveux.

On sait également que les phénomènes d'irritation provoqués par l'exposition aux rayons ultra-violet conduisent aussi au phénomène de vieillissement cellulaire accéléré, et sont actuellement considérés comme un facteur d'induction de tumeurs cutanées. L'irritation provoquée par le rayonnement UV donne lieu ici encore à la formation d'espèces radicalaires qui conduisent notamment à l'oxydation des lipides cutanés, et l'on pense que les peroxydes lipidiques sont l'un des facteurs déclenchants de la photocarcinogénèse. On sait en particulier que l'induction de l'ornithine décarboxylase (en abrégé: ODC) constitue un marqueur précoce des tumeurs de la peau, et que les peroxydes organiques sont capables d'induire la formation d'ODC dans l'épiderme ; voir R.L. Binder et al., Carcinogenesis, Vol.10, n°12, 2351-2357 (1989).

Les cellules vivantes possèdent divers moyens naturels de défense contre les peroxydes lipidiques, en particulier la glutathion peroxydase épidermique, mais l'efficacité de l'activité détoxifiante de cette dernière est fortement diminuée sous l'influence d'une exposition aux ultra-violets.

Il est donc important de mettre au point des systèmes actifs permettant de lutter contre les effets nocifs des peroxydes, notamment des peroxydes organiques formés sous l'action des polluants atmosphériques et des ultraviolets.

Il est connu que certains anti-oxydants sont susceptibles de conférer une protection contre les dommages cutanés causés par les radiations ou par les peroxydes, y compris lorsque ces antioxydants sont appliqués par voie topique ; voir par exemple Bissett et al., Photoderm. Photoimmunol. Photomed. 7,56-62 et 63-67 (1990).

En étudiant certains systèmes antioxydants et en utilisant l'induction d'ODC comme marqueur, la demanderesse a découvert que, de façon surprenante, certaines associations avaient la propriété d'inhiber la formation d'ODC alors que les constituants de l'association, utilisés seuls, étaient sans effet ou provoquaient même une augmentation de l'induction d'ODC.

On a découvert plus précisément que les peroxydases capables de réduire les peroxydes organiques provoquent une augmentation de l'induction d'ODC par les rayonnements ultra-violet, et que certains antioxydants sont sans effet significatif sur l'induction d'ODC. C'est le cas notamment des antioxydants capables de neutraliser l'oxygène singulet, qui sont donc des anti-oxygène singulet. On a toutefois découvert que l'association des peroxydases capables de réduire les peroxydes organiques avec les antioxydants capables de neutraliser l'oxygène singulet, permet de neutraliser de façon significative l'induction d'ODC. Une telle association est donc douée de propriétés de synergie.

Ces propriétés intéressantes peuvent être mises à profit par l'incorporation de telles associations synergiques dans des compositions cosmétiques ou pharmaceutiques sous une forme permettant l'application sur la peau, les phanères et les muqueuses.

L'invention a donc pour objet une composition cosmétique ou pharmaceutique caractérisée par le fait qu'elle comprend en association au moins un produit ayant une activité de peroxydase capable de réduire les peroxydes organiques et au moins un agent antioxydant capable de neutraliser l'oxygène singulet.

La composition de l'invention est une composition anti-oxydante, qui ne contient donc pas de peroxyde. En particulier, elle ne contient pas de peroxyde d'hydrogène.

On peut utiliser comme produit ayant une activité de peroxydase toute substance capable de réduire les peroxydes organiques en présence d'un donneur d'électrons.

Ces peroxydases peuvent être notamment des peroxydases d'origine naturelle (végétale ou animale), ou encore des peroxydases modifiées, chimiquement ou par greffage, par adsorption sur des supports, ou par encapsulation (voir par exemple les demandes PCT WO 87/07838 et EP-A-0 397 227).

On peut utiliser notamment les lactoperoxydases, les microperoxydases de champignons, la myéloperoxydase, etc...

On sait que la lactoperoxydase (en abrégé: LPO) est une enzyme que l'on trouve notamment dans de nombreux tissus et sécrétions de mammifères, qui utilise un des nombreux donneurs d'électrons cellulaires pour réduire les peroxydes organiques du type ROOH (R étant un groupement organique). La lactoperoxydase est un produit commercial, vendu notamment par les Sociétés Sigma et Sederma.

On peut également utiliser des peroxydases recombinantes, par exemple la LPO recombinante (demande de brevet WO 91-06639).

L'agent antioxydant capable de neutraliser l'oxygène singulet est choisi notamment parmi la quinoléine et ses dérivés, les polyphénols, les dérivés caroténoïdes et les nucléosides et leurs dérivés.

Parmi les dérivés de la quinoléine utilisables, on citera en particulier la 6-éthoxy 1,2-dihydro-2,2,4-triméthylquinoléine, ou éthoxyquine, sous forme de monomère, de dimère ou d'oligomère ou de mélanges de ces diverses formes, et les dérivés de l'éthoxyquine.

On peut utiliser en particulier les dérivés de l'éthoxyquine de formule (I) 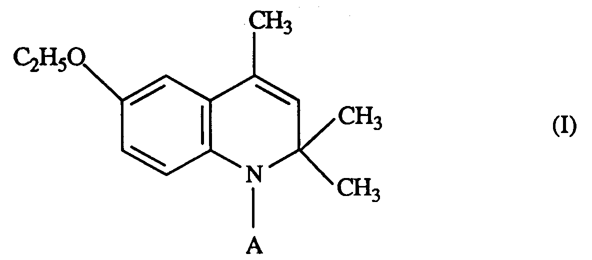 dans laquelle A représente un groupement -CO-B,
B représentant notamment un groupement (CHOR')ₙR dans lequel R' représente un atome d'hydrogène, un groupe acyle, alkyle ou aralkyle,
R est un groupement hydroxyméthyle, carboxyle, carboxyalkyle, carboxyaryle,
carboxyaryl-alkyle, carboxamide, -COR''', R''' représentant le reste de l'éthoxyquine (formule (I) moins le substituant A) ou R''' représentant -CH₂OR'', R'' étant un radical acyle, aralkyle ou alkyle et n étant un nombre entier de 2 à 6;
ou B représente un groupement -(CHR'''')ₘ N⁺R₁R₂R₃X⁻ dans lequel R'''' représente un atome d'hydrogène, un radical hétérocyclique, aralkyle, aryle, alkyle, éventuellement substitué, ou R'''' représente -(CH₂)_{q}COOH où q est un nombre pouvant varier de 1 à 3, R₁, R₂ et R₃ représentant indépendamment un atome d'hydrogène, un radical aryle, un radical aryle hétérocyclique, un radical cycloalkyle ou alkyle, substitué ou non, X⁻ étant un anion et m étant un nombre entier de 1 à 6;
ou B représente un groupement -(CHR'''')ₘNR₁R₂, où R'''', R₁, R₂ et m ont les significations mentionnées ci-dessus;
ou B représente un groupement NR'₁R'₂ dans lequel R'₁ et R'₂ représentent indépendamment un groupement aryle, aryle hétérocyclique, cycloalkyle ou alkyle, substitué ou non, ou bien R'₁ et R'₂ représentent -H ou forment un groupement hétérocyclique avec l'atome d'azote auquel ils sont attachés;
ou B représente un groupement -OR₄ dans lequel R₄ représente un groupement aryle, alkyle, polyhydroxyalkyle, cycloalkyle ou un groupement de formule 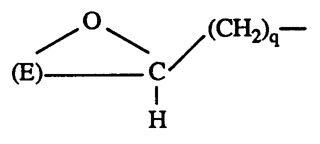
où E représente un groupement: 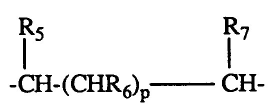
dans lequel R₅, R₆ et R₇ représentent indépendamment un atome d'hydrogène ou un radical -CH₂OH, -CH₂O acyle, -OH, -O acyle, -NH acyle, -NH₂, N⁺H₃X⁻, X⁻ étant défini comme précédemment, ou bien R₅, R₆ et R₇ représentent un groupement -COOR₈, R₈ représentant un atome d'hydrogène, un groupement aralkyle, aryle, cycloalkyle ou alkyle substitué ou non, p est un nombre égal à 1 ou 2 et q est égal à 0 ou 1;
ou B représente un atome d'halogène,
ou bien A représente un groupement : 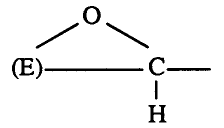
dans lequel E est défini comme précédemment.

De tels dérivés de l'éthoxyquine sont décrits notamment dans la demande de brevet FR-2.378.796.

Parmi les agents capables de neutraliser l'oxygène singulet, on citera également les polyphénols, c'est-à-dire les composés comportant au moins un cycle aromatique diphénolique, les groupements phénols pouvant être éventuellement étherifiés ou estérifiés. Parmi les polyphénols utilisables, on citera notamment les flavonoïdes répondant à la formule générale (II) : 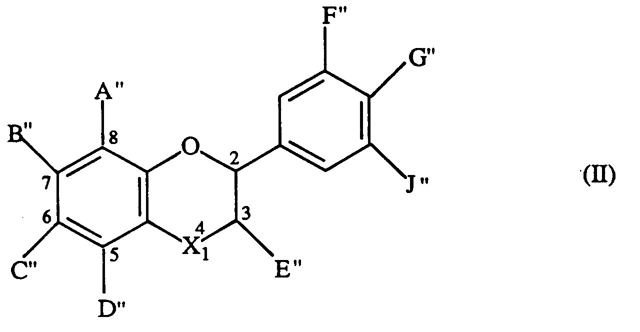
ou (III) : 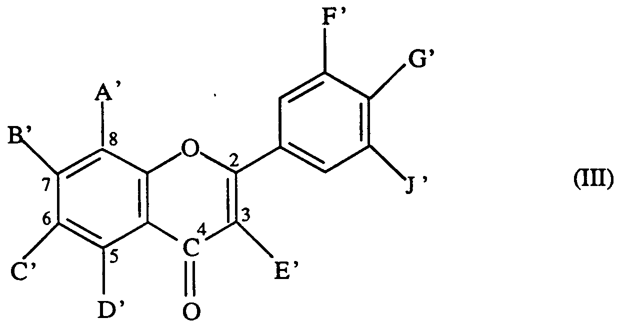 dans lesquelles A'', B'', C'', et D'', indépendamment l'un de l'autre, représentent H ou OH;
E'' représente H, OH ou OX', où X' représente: 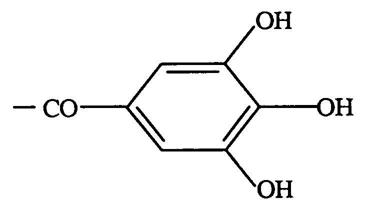
F'', G'', J'' représentent, indépendamment l'un de l'autre, H ou OH ; et X₁ représente -CH₂-, -CO- ou -CHOH-,
étant entendu qu'au moins deux des groupements A'', B'', C'' et D'' ou qu'au moins deux des radicaux F'', G'' et J'' désignent un groupe hydroxy,
A', C' et D', indépendamment l'un de l'autre, représentent H, OH ou OCH₃;
E' représente H, ou OR', où R' représente le reste d'un sucre de formule R'OH;
B', F', G' et J', indépendamment l'un de l'autre, représentent H, OH, OCH₃ ou -OCH₂-CH₂-OH, étant entendu qu'au moins deux des groupements A', B', C' et D' ne désignent pas -H ou qu'au moins un des groupements F', G' et J' ne désigne pas -H.

Parmi les sucres R'OH, on peut citer le rutinose.

Les composés de formule (II) et (III) sont connus. Ils peuvent être obtenus notamment selon les procédés décrits dans "The Flavonoids" Harborne J.B., Mabry T.J., Helga Mabry, 1975, pages 1 à 45.

Parmi les flavonoïdes utilisables selon l'invention, on citera notamment la taxifoline, la catéchine, l'épicatéchine, l'ériodictyol, la naringénine, la rutine, la troxérutine, la chrysine, la tangérétine, la lutéoline, l'épigallocatéchine et le gallate de l'épigallocatéchine, la quercétine, la fisétine, le kaëmpférol, la galangine, la gallocatéchine, le gallate d'épicatéchine.

De tels composés se trouvent notamment dans des extraits de thé vert vendus sous la dénomination Sunphenon par la Société Nikko.

Parmi les polyphénols utilisables, on citera également des polyphénols tels que l'acide carnosique et le carnosol qui peuvent être extraits par exemple du romarin soit par extraction suivie d'une distillation (Chang et al. JOSC, Vol.61, n°6, Juin 1984), soit par une extraction par un solvant polaire tel que l'éthanol précédée par une extraction à l'aide d'un solvant non polaire tel que l'hexane pour éliminer les substances odorantes, comme décrit dans la demande de brevet EP-307 626.

Les polyphénols utilisables peuvent également être choisis parmi les acides (2,5-dihydroxyphényl)alkylcarboxyliques de formule (IV) et leurs dérivés (notamment esters et amides): 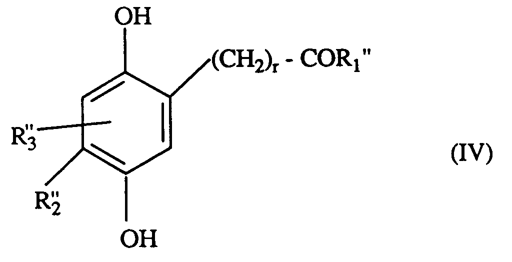
dans laquelle:
R''₁ représente -O-Alc, OH ou -N(r')(r"), Alc étant un alkyle linéaire ou ramifié en C₁-C₂₀, éventuellement substitué par un ou plusieurs groupements hydroxy ou alcoxy, ou Alc étant un alcényle en C₂-C₂₀,
r' et r'' représentent indépendamment H, alkyle C₁-C₂₀, hydroxyalkyle en C₂-C₆, ou polyhydroxyalkyle en C₃-C₆, ou bien r' et r'' forment ensemble, avec l'atome d'azote auquel ils sont rattachés, un hétérocycle,
r est un nombre, y compris zéro, tel que la chaîne -(CH₂)ᵣ-COR₁ comporte au plus 21 atomes de carbone,
R''₂ et R''₃ représentent indépendamment H ou un alkyle C₁-C₄, R''₂ pouvant représenter en outre un alcoxy C₁-C₄.

Les composés de formule (IV) sont connus ou peuvent être préparés selon des méthodes connues, par exemple analogues à celles décrites dans les brevets FR-2.400.358 et FR-2.400.359.

Parmi les polyphénols utilisables selon l'invention, on citera également les esters ou amides de l'acide caféique. Parmi les esters de l'acide caféique, on peut mentionner notamment les composés de formule (V): 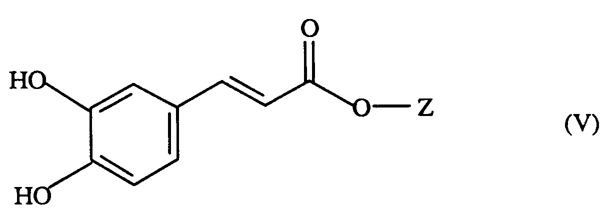
dans laquelle Z représente un alkyle C₁-C₈, par exemple méthyle, ou le reste d'un phytol.

Parmi les amides de l'acide caféique, on peut citer notamment les composés de formule (VI): 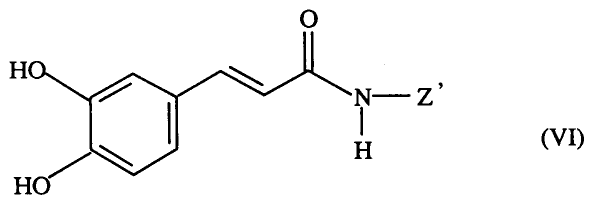
dans laquelle Z' représente un alkyle en C₁-C₈, en particulier en C₆-C₈.

Les composés de formule (V) ou (VI) sont connus ou peuvent être préparés selon les méthodes connues.

Parmi les antioxydants capables de neutraliser l'oxygène singulet, on citera également les dérivés caroténoïdes, et en particulier les composés suivants:
- All trans-betacarotène,
- alpha-carotène,
- gamma-carotène,
- delta-carotène,
- décapréno-beta-carotène,
- dodécapréno-beta-carotène,
- lycopène,
- zeaxanthine,
- astaxanthine,
- violaxanthine,
- lutéine,
- bixine,
- canthaxanthine,
- cryptoxanthine.

Parmi les agents antioxydants capables de neutraliser l'oxygène singulet, on citera également les nucléosides et leurs dérivés.

Les nucléosides (par exemple adénosine, guanosine, cytidine, thymidine et uridine et les dérivés de désoxyribose correspondants) sont notamment ceux dérivés de l'association d'une base purique ou pyrimidique choisie parmi l'adénine, la guanine, la cytosine, la thymine et l'uracile (en abrégé A, G, C, T, U) et d'un pentose (notamment ribose et désoxyribose). Les dérivés de nucléosides sont par exemple les mono-, di-, ou tri-phosphates, et notamment les 3'- et/ou 5'-phosphates, ainsi que les oligonucléotides ayant par exemple jusqu'à 20 motifs nucléotidiques.

Dans la composition de l'invention, la proportion pondérale de produit ayant une activité de peroxydase capable de réduire les peroxydes organiques peut varier de 0,005 % à 5 %, et en particulier de 0,01 % à 3 %.

La proportion pondérale d'agent antioxydant capable de neutraliser l'oxygène singulet peut varier par exemple de 0,005 % à 3 %, et en particulier de 0,01 % à 1 %.

Les proportions relatives de peroxydase et d'anti-oxygène singulet peuvent être déterminées dans chaque cas par de simples expériences de routine en sélectionnant les proportions relatives donnant des résultats favorables (synergie), par exemple dans le test d'induction de l'ODC décrit par R.L. Binder et al., article cité ci-dessus.

Généralement, le rapport pondéral peroxydase/anti-oxygène singulet peut varier par exemple de 0,001 à 0,3. Ce rapport est défini ici arbitrairement pour un produit à activité de peroxydase ayant une activité correspondant à 80 unités enzymatiques par mg. Il est donc facile d'adapter ce rapport dans le cas d'un produit à activité de peroxydase ayant un titre différent en unités enzymatiques. L'unité de peroxydase est définie ci-après dans la partie expérimentale.

Pour la réalisation des formes pharmaceutiques ou cosmétiques, selon les techniques connues, on tiendra compte évidemment des caractéristiques de solubilité des ingrédients, en liaison avec le type de composition souhaité.

Les produits ayant une activité de peroxydase réducteurs des peroxydes organiques, ainsi que les nucléosides et leurs dérivés, sont généralement solubles dans des phases hydrophiles, notamment aqueuses.

Les dérivés de quinoléine, les polyphénols, les caroténoïdes sont généralement solubles dans des phases lipophiles.

Les compositions de l'invention peuvent se présenter notamment sous la forme de solutions (compositions de type lotions), de solutions épaissies, de gels, de pommades, d'émulsions (crèmes, laits), de dispersions vésiculaires, de poudres, de poudres compactes, de pâtes ou de bâtonnets solides. Elles peuvent aussi être conditionnées, le cas échéant, dans des flacons pressurisés contenant un agent propulseur permettant l'application sous forme de mousses ou de sprays.

Les compositions cosmétiques ou pharmaceutiques de l'invention peuvent contenir, outre l'association de principes actifs décrite ci-dessus, les ingrédients ou adjuvants habituellement utilisés dans la réalisation de telles compositions, et en particulier des solvants tels que l'eau, des solvants organiques (par exemple alcools, huiles), ou des silicones, des agents épaississants, des agents tensioactifs, des polymères, des corps gras solides (par exemple cires, lanoline), des agents humectants, des agents conservateurs, des agents modificateurs de pH, des agents séquestrants, des agents colorants, des parfums, des charges solides (poudres et pigments), des substances absorbant l'ultra-violet, des agents autobronzants (comme la dihydroxyacétone), etc.

Les compositions sous forme de dispersions vésiculaires contiennent par exemple au moins un ingrédient actif incorporé dans des micelles ou des bicouches lipidiques, pouvant encapsuler une phase aqueuse, et dispersées dans un solvant aqueux.

Les dispersions vésiculaires de lipides, notamment de lipides amphiphiles ioniques ou non-ioniques, sont préparées selon des procédés connus, par exemple en faisant gonfler les lipides dans une solution aqueuse pour former des sphérules dispersées dans le milieu aqueux, comme décrit dans l'article de Banghan, Standish et Watkins, J. Mol. Biol. 13, 238 (1965) ou dans les brevets FR 2.315.991 et 2.416.008 de la demanderesse. On trouvera aussi la description de divers modes de préparation dans "Les liposomes en biologie cellulaire et pharmacologie". Edition Inserm/John Libbery Eurotext, 1987, pages 6 à 18.

La composition de l'invention peut contenir, en plus de l'association décrite ci-dessus, d'autres agents anti-oxydants tels que l'acide ascorbique, l'ascorbylphosphate de magnésium, les α, β, γ et/ou δ-tocophérols, la bilirubine, la biliverdine, les C₁-C₁₀ alkyl esters de glutathion, etc.

Les compositions de l'invention sont notamment des compositions cosmétiques ou pharmaceutiques protectrices de l'épiderme humain, des cheveux et des muqueuses, des compositions de maquillage de la peau et des phanères, des compositions à usages bucco-dentaires telles que des pâtes dentifrices, ou des compositions ophtalmiques telles que des collyres.

Lorsque la composition cosmétique selon l'invention est utilisée pour la protection des cheveux, elle peut se présenter sous forme de shampooings, de lotions, de gels ou de compositions à rincer, à appliquer avant ou après shampooing, avant ou après coloration ou décoloration, ou avant, pendant ou après traitement de permanente ou de défrisage. Elle peut encore se présenter sous la forme de lotions ou de gels coiffants ou traitants, de lotions ou gels pour le brushing ou la mise en plis, de laques pour cheveux, de compositions de permanente ou de défrisage, ou de compositions de coloration ou de décoloration des cheveux.

Lorsque la composition de l'invention est utilisée comme produit de maquillage des cils, des sourcils ou de la peau, elle se présente par exemple sous la forme de crèmes de traitement de l'épiderme, de fonds de teint, de bâtons de rouge à lèvres, de fards à paupières, de fards à joue, de ligneurs (encore appelés "eye-liners") ou de mascaras.

Les compositions de l'invention, et plus particulièrement les compositions de maquillage et les compositions solaires, peuvent contenir des pigments d'oxyde métallique tels que des oxydes de titane, de zinc, de cérium ou de zirconium, généralement à une concentration comprise entre 0,1 et 15 %, et en particulier entre 0,5 et 10 % en poids par rapport au poids total de la composition. Ces pigments sont utilisés de préférence sous la forme de nanopigments, de diamètre moyen inférieur à 100 nm, compris généralement entre 5 et 50 nm. Ces nanopigments peuvent éventuellement être enrobés. Les pigments enrobés sont des pigments qui ont suivi un ou plusieurs traitements de surface de nature chimique, électronique et/ou mécanique, avec des composés tels que des acides aminés, de la cire d'abeille, des acides gras, des alcools gras, des tensioactifs anioniques, des lécithines, des sels d'acides gras (sels de sodium, de potassium, de zinc, de fer ou d'aluminium), des alkoxydes métalliques (notamment de titane ou d'aluminium), du polyéthylène, des silicones, des protéines (par exemple collagène, élastine), des alcanolamines, des oxydes de silicium, des oxydes métalliques ou de l'hexamétaphosphate de sodium ; voir à ce sujet Cosmetics and Toiletries, Février 1990, Vol.105, pp.53-64.

Lorsque la composition de l'invention est une composition pharmaceutique, elle peut se présenter notamment sous forme d'émulsion (lait ou crème), de gel, de lotion, de pommade, de dispersion vésiculaire, et peut contenir, outre l'association décrite ci-dessus, un autre principe actif pharmaceutique.

Grâce à l'association synergique peroxydase + anti-oxygène singulet, les compositions de l'invention constituent des compositions cosmétiques ou pharmaceutiques destinées à être appliquées notamment sur la peau, les phanères et les muqueuses, qui permettent notamment de prévenir et traiter les dommages provoqués par les radicaux libres induits notamment par les polluants atmosphériques et par le rayonnement ultraviolet. En particulier, les compositions cosmétiques de l'invention permettent de prévenir ou de traiter notamment le phénomène de vieillissement accéléré de la peau. Les compositions de l'invention permettent en outre de prévenir ou de limiter les risques de cancers cutanés induits pas les ultraviolets.

L'un des avantages supplémentaires de l'association antioxydante selon l'invention est qu'elle permet d'inhiber ou de diminuer la réaction photoinduite qui apparait lorsque des pigments d'oxydes métalliques sont exposés à la lumière, et qui est préjudiciable à la stabilité des compositions, en particulier lorsque celles-ci contiennent aussi des lipides.

L'invention a également pour objet l'utilisation, en association, d'au moins un produit à activité de peroxydase capable de réduire les peroxydes organiques et d'au moins un antioxydant capable de neutraliser l'oxygène singulet, comme association active synergique dans la préparation d'une composition cosmétique ou pharmaceutique destinée à prévenir ou traiter les dommages cellulaires provoqués par les radicaux libres induits notamment par les polluants atmosphériques et/ou par le rayonnement ultraviolet, et/ou destinée à lutter contre le phénomène de vieillissement accéléré de la peau, ou à prévenir ou limiter les risques de tumeurs cutanées photo-induites.

L'invention a également pour objet un procédé de traitement cosmétique permettant de lutter contre les dommages esthétiques provoqués sur la peau et les cheveux par les radicaux libres induits notamment par les polluants atmosphériques et par le rayonnement ultraviolet, caractérisé par le fait que l'on applique sur la peau ou les cheveux, une composition contenant l'association synergique qui a été décrite ci-dessus.

Les exemples suivants illustrent l'invention.

Dans ces exemples, l'origine ou la nature des ingrédients est la suivante:
- Lactoperoxydase (en abrégé LPO) : sous la forme de poudre; origine Société Sederma (France).
- Ethoxyquine: mélange de monomère, dimère et polymères de la 6-éthoxy-1,2-dihydro-2,2,4-triméthylquinoléine commercialisé sous la dénomination Santoquin par la Société Monsanto
- Guanosine : origine Pharma Waldhol
- Composé A : composé amphiphile non ionique de formule: 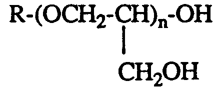 dans laquelle R est un radical hexadécyle et
   n a une valeur statistique moyenne égale à 3.
- Hydroviton: mélange d'amino-acides, de principes hydratants de la peau et de lactate de sodium, d'allantoïne (tampon), commercialisé par Dragoco
- Cire de Sipol : alcool cétylstéarylique partiellement oxyéthyléné, commercialisé par Sinnova-France
- Carbopols : polymères carboxyvinyliques réticulés commercialisés par Goodrich.

### EXEMPLE 1: Emulsion H/E

| | |
|---|---|
| - Lactoperoxydase (LPO) | 0,05 % |
| - Ethoxyquine | 0,26 % |
| - Monostéarate de polyéthylèneglycol 50 OE (ICI) | 1,5 % |
| - Mélange de mono- et distéarate de diglycérol (stéarineries Dubois, France) | 1,5 % |
| - Huile de vaseline | 24 % |
| - Alcool cétylique | 2,5 % |
| - Eau q.s.p. | 100 % |

Cette émulsion constitue une crème. Pour la préparer, on verse la phase aqueuse dans la phase grasse, à 80°C, sous agitation.

Cette composition est appliquée sur le visage.

### EXEMPLE 2: Fluide de soin pour le corps

On prépare d'abord de la façon connue un support sous la forme d'une dispersion de sphérules lipidiques, de composition suivante (en % par rapport à la composition finale) :

| | |
|---|---|
| - Composé A | 4,5 % |
| - Cholestérol | 4,5 % |
| - Dicétylphosphate | 1,0 % |
| - Parahydroxybenzoate de méthyle | 0,3 % |
| - Eau déminéralisée stérile | 30 % |

Pour cela, on mélange par fusion à 100°C les trois premiers ingrédients, sous atmosphère d'azote, on refroidit à 80°C puis homogénéise à l'aide d'un ultradisperseur de type Virtis. On ajoute ensuite l'eau et l'agent conservateur. La dispersion est ramenée à la température ambiante et on y ajoute 0,1 % de lactoperoxydase, puis la phase A ci-après:

### Phase A:

| | |
|---|---|
| - Parfum | 0,4 % |
| - Huile de tournesol | 10 % |
| - Huile de paraffine | 4 % |
| - Vitamine F | 2 % |
| - Lécithine de soja | 1 % |
| - Palmitate d'ascorbyle | 1 % |
| - Salicylate d'hexadécylamine | 0,2 % |
| - Ethoxyquine | 0,8 % |

On homogénéise à l'aide d'un ultradisperseur; on disperse ensuite la phase B constituée de:

| | |
|---|---|
| - "Carbopol 940" | 0,4 % |
| - Eau déminéralisée | 79,7 % |

L'ensemble est enfin neutralisé à l'aide de 0,4 % de triéthanolamine.

### EXEMPLE 3: Lait de beauté pour le corps

On a préparé une émulsion huile-dans-l'eau (H/E) de composition suivante :

| | |
|---|---|
| - Huile de purcellin (Dragoco) | 2 g |
| - Huile de vaseline | 6 g |
| - Alcool oléique | 1 g |
| - Myristate d'isopropyle | 1,5 g |
| - Monostéarate de glycérine | 2 g |
| - Stéarine | 1,4 g |
| - Alcool cétylique | 0,1 g |
| - Parfum | 0,9 g |
| - Carbopol 941 | 0,35 g |
| - Triéthanolamine pure | 1,05 g |
| - Parahydroxybenzoate de butyle | 0,04 g |
| - Agent conservateur | 0,3 g |
| - Propylèneglycol | 5 g |
| - Hydroviton | 1,5 g |
| - Guanosine | 0,5 g |
| - Colorant F.D.C. blue 1 (Kohnstamn) à 1 % dans l'eau | 0,03 g |
| - Lactoperoxydase | 0,1 g |
| - Eau déminéralisée | 71,55 g |

### EXEMPLE 4: Crème pour le corps

On a préparé une émulsion H/E de composition suivante :

| | |
|---|---|
| - Alcool cétylique | 0,5 g |
| - Cire de sipol | 5 g |
| - Monostéarate de glycérol | 1,5 g |
| - Huile de vaseline | 6 g |
| - Myristate d'isopropyle | 3 g |
| - Glycérine | 10 g |
| - Parfum | 0,2 g |
| - Guanosine | 1,5 g |
| - Lactoperoxydase | 0,2 g |
| - Eau q.s.p. | 100 g |

### EXEMPLE 5: Crème pour le corps

On a préparé une émulsion H/E de composition suivante :

| | |
|---|---|
| - Cire de Sipol | 6 g |
| - Monstéarate de glycérol | 1,5 g |
| - Stéarate de sodium | 0,8 g |
| - Huile de vaseline | 6 g |
| - Palmitate d'isopropyle | 2 g |
| - Ethoxyquine | 1 g |
| - Glycérine | 15 g |
| - Parfum | 0,3 g |
| - Lactoperoxydase | 2 g |
| - Eau q.s.p. | 100 g |

### EXEMPLE 6: Crème dermatopharmaceutique

### Phase A:

| | |
|---|---|
| - Stéarate de polyéthylène glycol vendu sous la dénomination "Myrj 49" par la Société ICI | 1,75 g |
| - Stéarate de glycérol et stéarate de polyéthylène glycol vendu sous la dénomination "Arlacel 165" par la Société ICI | 1,75 g |
| - Alcool cétylique | 0,6 g |
| - Alcool stéarylique | 0,6 g |
| - Huile de vaseline | 17 g |
| - Acide stéarique | 2,5 g |

### Phase B :

| | |
|---|---|
| - Acide polyacrylique réticulé vendu sous la dénomination "Carbopol 941" par la Société Goodrich | 0,4 g |
| - Lactoperoxydase | 0,1 g |
| - Glycérine | 3 g |
| - Parahydroxybenzoate de méthyle | 0,1 g |
| - Sel tétrasodique de l'acide éthylène-diamine tétracétique | 0,1 g |
| - Triéthanolamine (solution aqueuse à 20%) | 0,5 g |
| - Eau | 66,53 g |

### Phase C:

| | |
|---|---|
| - Ethoxyquine | 0,05 g |
| - Acide rétinoïque | 0,02 g |
| - Myristate d'isopropyle | 5 g |

On mélange à 70°C, les constituants de la phase grasse A.

On solubilise sous agitation, les constituants de la phase aqueuse B, chauffée à 70°C.

On ajoute sous agitation la phase aqueuse B dans la phase grasse A. On ajoute à l'émulsion obtenue l'éthoxyquine et l'acide rétinoïque solubilisés dans le myristate d'isopropyle *(Phase C).*

On obtient une crème de soin anti-vieillissement qui peut être appliquée sur le visage.

### EXEMPLE 7: Gel dermatopharmaceutique anti-rides

On mélange, à température ambiante et sous agitation, les composés suivants :

| | |
|---|---|
| - Symperonic PE/L62* | 0,2 g |
| - Propylène glycol | 4 g |
| - Ethoxyquine | 0,1 g |
| - Acide lactique | 1 g |
| - Sel tétrasodique de l'acide éthylènediamine tétracétique | 0,1 g |
| - Lactoperoxydase | 0,4 g |
| - Phénoxyéthanol | 0,25 g |
| - Eau q.s.p. | 100 g |

| | |
|---|---|
| *commercialisé par la Société ICI : polymère séquencé polyoxyéthylène/polyoxypropylène/polyoxyéthylène:Poloxamer 182 (CTFA). | |

On ajoute à la dispersion obtenue 1 g d'acide polyacrylique réticulé vendu sous la dénomination Carbopol 940 par la Société Goodrich, puis on ajoute de la soude pour ajuster le pH à 5.

Le gel obtenu est appliqué sur le visage et le cou.

### EXEMPLE 8: Crème de soin protectrice

| | |
|---|---|
| - "Sinnowax AO" | 5 g |
| - Stéarate de glycérol | 1 g |
| - Alcool cétylique | 1 g |
| - Huile de jojoba | 6 g |
| - Acide linoléique | 6 g |
| - "MT 100 T"(TiO₂) | 5 g |
| - Ethoxyquine | 1 g |
| - Lactoperoxydase | 0,04 g |
| - Conservateurs q.s. | |
| - Eau q.s.p. | 100 g |

On chauffe la phase grasse à 80°C. On ajoute l'oxyde de titane.

On verse la phase aqueuse, sous agitation à 80°C, dans la phase grasse.

Cette composition sous forme de crème est appliquée sur le visage.

Sinnowax AO, commercialisé par la Société Henkel, est un mélange d'alcool cétylstéarylique et d'alcool cétylstéarylique oxyéthyléné à 33 moles d'oxyde d'éthylène.

MT 100 T est la dénomination commerciale d'un oxyde de titane commercialisé par la Société Tayca.

### ETUDE PHARMACOLOGIQUE

Le test utilisé consiste à évaluer l'induction de la formation d'ODC par irradiation selon une technique analogue à celle décrite par R.L. Binder et al., article cité ci-dessus.

Les produits à évaluer sont étudiés sous forme de crème, contenant 30 u/g de lactoperoxydase et 1 % d'éthoxyquine.

On rappelle qu'une unité de lactoperoxydase forme 1 mg de purpurogalline à partir du pyrogallol en 20 secondes (20°C) à pH 6.0. La formule à 30 u/g contient 0,039 % en poids de lactoperoxydase.

On a comparé cette crème à une crème ne contenant que l'éthoxyquine, ou ne contenant que la lactoperoxydase, à la même concentration.

### Résultats

L'éthoxyquine seule est sans effet significatif sur la formation d'ODC, par comparaison avec un placebo. La lactoperoxydase seule conduit à une augmentation importante (72 % dans le cas présent) de la formation d'ODC, par rapport à un placebo.

Par contre, l'association de ces deux ingrédients conduit à une diminution importante (-54 % dans le cas présent) de la formation d'ODC par rapport au placebo.

## Revendications

1. Composition cosmétique ou pharmaceutique, caractérisée par le fait qu'elle comprend en association au moins un produit ayant une activité de peroxydase capable de réduire les peroxydes organiques et au moins un agent antioxydant capable de neutraliser l'oxygène singulet, ladite composition étant exempte de peroxyde.

2. Composition selon la revendication 1, caractérisée par le fait que ledit produit à activité de peroxydase est choisi parmi la lactoperoxydase, les microperoxydases de champignons et la myéloperoxydase.

3. Composition selon l'une quelconque des revendications précédentes, caractérisée par le fait que la proportion pondérale de produit ayant une activité de peroxydase capable de réduire les peroxydes organiques est dans la gamme de 0,005 % à 5 % et en particulier de 0,01% à 3 %.

4. Composition selon l'une quelconque des revendications précédentes, caractérisée par le fait que l'agent antioxydant capable de neutraliser l'oxygène singulet est choisi parmi la quinoléine et ses dérivés, les polyphénols, les dérivés caroténoïdes et les nucléosides et leurs dérivés.

5. Composition selon la revendication 4, caractérisée par le fait que le dérivé de quinoléine est choisi parmi l'éthoxyquine, sous forme de monomère, de dimère ou d'oligomère, de mélanges de ces diverses formes, et les dérivés de l'éthoxyquine.

6. Composition selon la revendication 4, caractérisée par le fait que lesdits polyphénols comprennent les flavonoïdes, les polyphénols extraits du romarin, les acides (2,5-dihydroxyphényl)alkylcarboxylique et leurs dérivés, notamment esters et amides tels que les esters ou amides de l'acide caféique.

7. Composition la revendication 4, caractérisée par le fait que lesdits nucléosides et leurs dérivés sont choisis parmi les osides dérivés des bases puriques ou pyrimidiques, les mono-, di- et tri-phosphates correspondants et les oligonucléotides ayant jusqu'à 20 motifs nucléotidiques.

8. Composition selon la revendication 4, caractérisée par le fait que ledit agent antioxydant est la guanosine.

9. Composition selon l'une quelconque des revendications précédentes, caractérisée par le fait que la proportion pondérale d'agent antioxydant capable de neutraliser l'oxygène singulet est dans la gamme de 0,005 % à 3 % et en particulier de 0,01 % à 1 %.

10. Composition selon l'une quelconque des revendications précédentes, caractérisée par le fait que le rapport pondéral du produit à activité de peroxydase à l'antioxydant capable de neutraliser l'oxygène singulet est dans la gamme de 0,001 à 0,3, ce rapport étant défini arbitrairement pour un produit à activité de peroxydase ayant une activité correspondant à 80 unités enzymatiques par mg.

11. Composition selon l'une quelconque des revendications précédentes, caractérisée par le fait qu'elle se présente sous la forme d'une solution épaissie, d'un gel, d'une pommade, d'une émulsion, d'une dispersion vésiculaire, d'une poudre compacte, d'une pâte ou d'un bâtonnet solide.

12. Composition selon l'une quelconque des revendications précédentes, caractérisée par le fait qu'elle constitue une composition protectrice de l'épiderme humain, des cheveux et des muqueuses, une composition de maquillage de la peau et des phanères, une composition à usage bucco-dentaire ou une composition ophtalmique telle qu'un collyre.

13. Composition selon l'une quelconque des revendications précédentes, caractérisée par le fait qu'elle contient des pigments d'oxydes métalliques.

14. Utilisation, en association, d'au moins un produit à activité de peroxydase capable de réduire les peroxydes organiques et d'au moins un antioxydant capable de neutraliser l'oxygène singulet, comme association active synergique dans la préparation d'une composition cosmétique ou pharmaceutique destinée à prévenir ou traiter les dommages cellulaires provoqués par les radicaux libres induits notamment par les polluants atmosphériques et/ou par le rayonnement ultraviolet, et/ou destinée à lutter contre le phénomène de vieillissement accéléré de la peau, ou à prévenir ou limiter les risques de cancers cutanés photo-induits.

15. Procédé de traitement cosmétique permettant de lutter contre les dommages esthétiques provoqués sur la peau et les cheveux par les radicaux libres induits notamment par les polluants atmosphériques et le rayonnement ultraviolet, caractérisé par le fait que l'on applique sur la peau ou les cheveux une composition telle que définie dans l'une quelconque des revendications 1 à 13.

## Claims

1. Cosmetic or pharmaceutical composition, characterized by the fact that it comprises, in combination, at least one product having a peroxidase activity capable of reducing organic peroxides and at least one antioxidant capable of neutralizing singlet oxygen, the said composition being free of peroxide.

2. Composition according to Claim 1, characterized by the fact that the said product with peroxidase activity is chosen from lactoperoxidase, fungal microperoxidases and myeloperoxidase.

3. Composition according to any one of the preceding claims, characterized by the fact that the proportion by weight of product having a peroxidase activity capable of reducing organic peroxides is in the range of 0.005 % to 5 %, and in particular of 0.01 % to 3 %.

4. Composition according to any one of the preceding claims, characterized by the fact that the antioxidant capable of neutralizing singlet oxygen is chosen from quinoline and its derivatives, polyphenols, carotenoid derivatives and nucleosides and their derivatives.

5. Composition according to Claim 4, characterized by the fact that the quinoline derivative is chosen from ethoxyquine, in the form of a monomer, dimer or oligomer, mixtures of these various forms, and ethoxyquine derivatives.

6. Composition according to Claim 4, characterized by the fact that the said polyphenols comprise flavonoids, polyphenols extracted from rosemary, (2,5-dihydroxyphenyl)alkylcarboxylic acids and their derivatives, especially esters and amides such as the esters or amides of caffeic acid.

7. Composition according to Claim 4, characterized by the fact that the said nucleosides and their derivatives are chosen from glycosides derived from purine or pyrimidine bases, the corresponding mono-, di- and triphosphates and the oligonucleotides having up to 20 nucleotide units.

8. Composition according to Claim 4, characterized by the fact that the said antioxidant is guanosine.

9. Composition according to any one of the preceding claims, characterized by the fact that the proportion by weight of antioxidant capable of neutralizing singlet oxygen is in the range of 0.005 % to 3 %, and in particular of 0.01 % to 1 %.

10. Composition according to any one of the preceding claims, characterized by the fact that the weight ratio of the product with peroxidase activity to the antioxidant capable of neutralizing singlet oxygen is in the range of 0.001 to 0.3, this ratio being arbitrarily defined for a product with peroxidase activity having an activity corresponding to 80 enzymatic units per mg.

11. Composition according to any one of the preceding claims, characterized by the fact that it is provided in the form of a thickened solution, a gel, an ointment, an emulsion, a vesicular dispersion, a dense powder, a paste or a solid stick.

12. Composition according to any one of the preceding claims, characterized by the fact that it constitutes a composition for protecting the human epidermis, the hair and the mucous membranes, a makeup composition for the skin and the superficial body growths, a composition for buccodental use or an ophthalmic composition such as a collyrium.

13. Composition according to any one of the preceding claims, characterized by the fact that it contains pigments of metallic oxides.

14. Use, in combination, of at least one product with peroxidase activity capable of reducing organic peroxides and of at least one antioxidant capable of neutralizing singlet oxygen, as synergistic active combination in the preparation of a cosmetic or pharmaceutical composition intended to prevent or treat the cellular damage caused by the free radicals induced especially by atmospheric pollutants and/or by ultraviolet radiation, and/or intended to combat the phenomenon of accelerated aging of the skin, or to prevent or limit the risks of photoinduced skin cancers.

15. Cosmetic treatment process which makes it possible to combat the aesthetic damage caused to the skin and the hair by the free radicals induced especially by atmospheric pollutants and by ultraviolet radiation, characterized by the fact that a composition as defined in any one of Claims 1 to 13 is applied to the skin or the hair.

## Patentansprüche

1. Kosmetische oder pharmazeutische Zubereitung, dadurch gekennzeichnet, daß sie in Kombination mit mindestens einem Produkt mit Peroxidaseaktivität, das organische Peroxide reduzieren kann, mindestens ein Antioxidans enthält, das Singulett-Sauerstoff neutralisieren kann, wobei die Zubereitung frei von Peroxid ist.

2. Zubereitung gemäß Anspruch 1, dadurch gekennzeichnet, daß das Produkt mit Per oxidaseaktivität aus Lactoperoxidase, den Mikroperoxidasen aus Pilzen und der Myeloperoxidase ausgewählt ist.

3. Zubereitung gemäß einem der vorstehenden Ansprüche, dadurch gekennzeichnet, daß der Gewichtanteil des Produkts mit Peroxidaseaktivität, das organische Peroxide reduzieren kann, im Bereich von 0,005 und 5 %, insbesondere von 0,01 % bis 3 % liegt.

4. Zubereitung gemäß einem der vorstehenden Ansprüche, dadurch gekennzeichnet, daß das Antioxidans, das Singulett-Sauerstoff neutralisieren kann, aus Chinolin und dessen Derivaten, den Polyphenolen, den Carotinoiden sowie den Nukleosiden und deren Derivaten ausgewählt ist.

5. Zubereitung gemäß Anspruch 4, dadurch gekennzeichnet, das das Chinolinderivat aus Ethoxychinolin in Form des Monomeren, Dimeren oder der Oligomeren bzw. aus Gemischen dieser verschiedenen Formen sowie den Ethyoxychinolinderivaten ausgewählt ist.

6. Zubereitung gemäß Anspruch 4, dadurch gekennzeichnet, daß die genannten Polyphenole die Flavonoide, Polyphenolextrakte aus Rosmarin, (2,5-Dihydroxyphenyl)alkylcarbonsäuren und deren Derivate, insbesondere Ester und Amide sowie die Kaffeesäureester und -amide umfassen.

7. Zubereitung gemäß Anspruch 4, dadurch gekennzeichnet, daß die genannten Nukleoside und deren Derivate aus den von Purin- und Pyrimidinbasen abgeleiteten Glykosiden, den entsprechenden Mono-, Di- und Triphosphaten sowie Oligonukleotiden mit bis zu 20 Nukleotideinheiten ausgewählt sind.

8. Zubereitung gemäß Anspruch 4, dadurch gekennzeichnet, daß es sich bei dem Antioxidans um Guanosin handelt.

9. Zubereitung gemäß einem der vorstehenden Ansprüche, dadurch gekennzeichnet, daß der Gewichtsanteil des Antioxidans, das den Singulett-Sauerstoff neutralisieren kann, im Bereich von 0,005 % und 3 %, insbesondere von 0,01 % bis 1 % liegt.

10. Zubereitung gemäß einem der vorstehenden Ansprüche, dadurch gekennzeichnet, daß das Gewichtsverhältnis des Produktes mit Peroxidaseaktivität zum Antioxidans, das den Singulett-Sauerstoff neutralisieren kann, zwischen 0,001 und 0,3 liegt, wobei dieses Verhältnis willkürlich für ein Produkt mit einer Peroxidaseaktivität, entsprechend 80 Enzymeinheiten pro mg, definiert ist.

11. Zubereitung gemäß einem der vorstehenden Ansprüche, dadurch gekennzeichnet, daß das es in Form einer verdickten Lösung, eines Gels, einer Salbe, einer Emulsion, einer vesikulären Dispersion, eines Kompaktpuders, einer Paste oder eines festen Sticks (in Stäbchenform) vorliegt.

12. Zubereitung gemäß einem der vorstehenden Ansprüche, dadurch gekennzeichnet, daß es sich um eine die menschliche Haut, die Haare oder die Schleimhäute schützende Zubereitung, eine Zubereitung zum Schminken von Haut und Nägeln oder anderer Epidermisbildungen, eine Zubereitung zur Verwendung bei der Mund- oder Zahnhygiene oder eine ophthalmologische Zubereitung wie Augentropfen handelt.

13. Zubereitung gemäß einem der vorstehenden Ansprüche dadurch gekennzeichnet, daß sie Metalloxidpigmente enthält.

14. Verwendung mindestens eines Produkts mit Peroxidaseaktivität, das organische Peroxide reduzieren kann, in Kombination mit mindestens einem Antioxidans, das Singulett-Sauerstoff neutralisieren kann, als synergistische Kombination zur Herstellung einer kosmetischen oder Pharmazeutischen Zubereitung zur Vorbeugung oder zur Behandlung von durch freie Radikale hervorgerufenen Zellschäden, die insbesondere durch Luftverschmutzung und/oder ultraviolene Bestrahlung induziert werden, und/oder zur Bekämpfung des Phänomens der beschleunigten Hautalterung oder zur Vorbeugung gegen das Risiko oder Begrenzung des Risikos von photoinduziertem Hautkrebs.

15. Kosmetisches Behandlungsverfahren, das die Bekämpfung von auf der Haut oder den Haaren durch freie Radikale hervorgerufenen ästhetischen Beeinträchtigungen, insbesondere durch Luftverschmutzung und UV-Bestrahlung hervorgerufener Beeinträchtigungen ermöglicht, dadurch gekennzeichnet, daß man auf die Haut oder die Haare eine Zubereitung gemäß einem der Ansprüche 1 bis 13 aufträgt.
